Europäisches Patentamt

**European Patent Office**

Office européen des brevets

(19)

(11) Numéro de publication: **0 240 373**

**A1**

(12) # DEMANDE DE BREVET EUROPEEN

(21) Numéro de dépôt: **87400206.6**

(22) Date de dépôt: **29.01.87**

(51) Int. Cl.³: **C 12 N 15/00**

(30) Priorité: **31.01.86 FR 8601391**

(43) Date de publication de la demande:
**07.10.87 Bulletin 87/41**

(84) Etats contractants désignés:
**AT BE CH DE ES FR GB GR IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Cuzin, Francois**
**Villa Orangini 119 avenue de Brancelar**
**F-06000 Nice(FR)**

(72) Inventeur: **Leopold, Pierre**
**17, rue de la Préfecture**
**F-06300 Nice(FR)**

(72) Inventeur: **Rassoulzadegan, Minoo**
**91 avenue des Arennes de Cimiez**
**F-06000 Nice(FR)**

(72) Inventeur: **Vailly, Joelle**
**72 avenue Bieckert**
**F-06000 Nice(FR)**

(74) Mandataire: **Peaucelle, Chantal et al,**
**S.C. Ernest Gutmann - Yves Plasseraud 67, boulevard Haussmann**
**F-75008 Paris(FR)**

(54) Fragments d'ADN utilisables dans des vecteurs pour le maintien héréditaire de gènes étrangers à l'état autonome dans des animaux transgéniques, procédé pour leur obtention et applications biologiques.

(57) Les fragments d'ADN de l'invention renferment des séquences de nucléotides à fonction centromérique.

EP 0 240 373 A1

FRAGMENTS D'ADN UTILISABLES DANS DES VECTEURS POUR LE MAINTIEN HEREDITAIRE DE GENES ETRANGERS A L'ETAT AUTONOME DANS DES ANIMAUX TRANSGENIQUES, PROCEDE POUR LEUR OBTENTION ET APPLICATIONS BIOLOGIQUES.

-----------------------------------------------------

L'invention a pour objet des fragments d'ADN utilisables dans des vecteurs pour la transmission et le maintien de gènes étrangers dans des animaux transgéniques. Elle vise également un procédé pour leur obtention et leurs applications biologiques.

On sait que les animaux peuvent être génétiquement transformés en insérant des gènes clonés dans leur génome.

L'intérêt pratique et économique de l'établissement de lignées d'animaux transgéniques est considérable. Les animaux peuvent être en effet transformés génétiquement pour améliorer certaines de leurs caractéristiques, telles que la résistance aux maladies, la croissance, la production d'hormones, de produits alimentaires ou encore la capacité d'adaptation à un autre environnement.

Les techniques connues d'insertion de gènes chez les mammifères sont basées sur l'injection directe d'un gène cloné dans un jeune embryon on fait intervenir un vecteur viral pour introduire le gène dans des cellules embryonnaires. Pour que le gène se transmette de génération en génération, on effectue son insertion dans les lignées germinales. Cependant, avec les moyens utilisés à ce jour, l'ADN injecté dans l'oeuf fertilisé s'est toujours intégré dans le chromosome de l'hôte transgénique, ce qui présente de nombreux inconvénients.

En effet, le lieu d'insertion est aléatoire l'intégration entraîne de nombreux remaniements dans le génome, le taux de transmission n'est pas constant et dépend du site d'intégration qui lui-même n'est pas maîtrisé.

L'invention a pour but de remédier au moins en partie aux inconvénients des techniques de l'art antérieur à l'aide de moyens permettant le transfert de gènes étrangers sous forme de vecteurs autonomes particuliers.

Dans des travaux précédents, les inventeurs ont obtenu des plasmides circulaires autonomes par microinjection dans des oeufs de souris fécondés de molécules circulaires du plasmide pPyLT1. Il s'agit d'un plasmide comprenant le gène codant pour la protéine grand T du virus du polyome et des séquences bactériennes vectrices dérivées de pBR322. Les plasmides autonomes ont été maintenus de façon stable en un petit nombre de copies dans des souches transgéniques et contenaient suffisamment de séquences de pBR322 intactes pour pouvoir être récupérées et amplifiées dans E. COLI.

L'état d'avancement des travaux des inventeurs dans ce domaine leur ont permis de mettre en évidence que de tels plasmides avaient acquis des séquences génomiques cellulaires définies utilisables pour la construction de vecteurs de microinjection.

L'invention a donc pour but de fournir de nouveaux fragments d'ADN correspondant à ou comprenant de telles séquences.

Elle vise également à fournir ces fragments d'ADN associés à un ou plusieurs gènes d'intérêt économique ou scientifique, en particulier sous forme de vecteurs.

L'invention a également pour but de fournir un procédé d'obtention de ces fragments, et de construction de vecteurs les renfermant, basé d'une manière avantageuse sur les techniques classiques de génie génétique.

Selon un autre aspect, l'invention vise également les applications biologiques de ces fragments et vecteurs, en particulier leur utilisation pour l'élaboration de vecteur de microinjection permettant notamment l'établissement de lignées d'animaux transgéniques.

Les fragments d'ADN de l'invention sont caractérisés en ce qu'ils renferment une séquence de nucléotides à fonction centromérique, ou séquence cen, capable d'assurer la ségrégation régulière lors des mitoses et des méioses, associée à des séquences ori nécessaires à la réplication à l'état autonome.

Des fragments d'ADN à fonction homologue peuvent être obtenus par clonage à partir de l'ADN cellulaire de vertébrés, notamment d'animaux d'élevage, en utilisant comme sondes les séquences obtenues par transposition chez la souris.

Ces fragments d'ADN comprennent ou correspondent à des fragments d'ADN tels qu'obtenus par transposition d'ADN cellulaire _____ de souris, dans un vecteur circulaire à la suite de l'expression d'un antigène T de Polyomavirus.

D'une manière avantageuse, ces fragments peuvent être insérés dans des vecteurs en association avec un ou plusieurs gènes étrangers à un animal donné, présentant un intérêt économique ou scientifique.

Les fragments d'ADN insérés correspondent aux fragments d'ADN définis ci-dessus, ou en variante à des fragments de ce type comportant, à l'une ou aux deux extrémités, des sites de restriction naturels et/ou artificiellement ajoutés (linkers).

Les fragments d'ADN comportant ou correspondant à la séquence cen, encadrés par les sites de restriction ou comportant un de ces sites à une extrémité entrent également dans le cadre de l'invention. Ils

4

constituent un module centromérique pourvu d'un système d'accrochage.

Les sites de restriction ajoutés sont choisis parmi ceux compatibles avec la fonction de segrégation et/ou de réplication du fragment cen. Ces sites doivent également permettre la coupure par une endonucléose de restriction et la soudure à un fragment d'ADN donné.

Des exemples de linkers sont donnés notamment par Norrander et al dans Gene 26,101-106, 1983.

L'invention vise également des vecteurs circulaires autonomes comprenant un fragment d'ADN avec la séquence cen, des sites de restriction naturels et/ou artificiellement ajoutés, un ou plusieurs gènes présentant un intérêt économique ou scientifique et une séquence vectrice.

Les gènes insérés sont avantageusement choisis parmi les gènes capables d'améliorer une race animale d'un point de vue économique. Il s'agit notamment de gènes de résistance aux maladies, de gènes permettant d'augmenter le niveau de production d'une hormone, d'un produit alimentaire comme le lait, ou encore d'un produit utilisable dans l'industrie tel que la laine. On peut également utiliser un gène permettant d'effectuer une étude fondamentale.

La séquence vectrice utilisée est capable de jouer le rôle de navette entre l'animal transgénique et un hôte constitué plus spécialement par une bactérie, telle que E. coli, ou une levure telle que S.cerevisiae.

Comme exemple de séquences vectrices appropriées, on citera des séquences plasmidiques par exemple de pBR322 et dérivés, YEp13 ou analogues ou encore des séquences de rétrovirus.

La propriété de navette entre l'animal et l'hôte présente un grand intérêt en permettant de vérifier la structure du vecteur transgénique, sa stabilité, d'effectuer une sélection au niveau de l'animal pour un phénotype intéressant.

L'animal est avantageusement un animal d'élevage tel que vache, mouton, porc, lapin, poule ou autres.

Conformément à l'invention, les fragments d'ADN définis ci-dessus sont isolés par clonage à partir de vecteurs circulaires tels qu'obtenus à la suite de l'expression d'un antigène T de polyomarivus.

En opérant selon les techniques classiques du génie génétique, on a recours à un procédé comprenant :
- la définition d'une carte de restriction du vecteur,
- l'identification des fragments comportant des séquences cellulaires, par exemple par hybridation,
- la purification du fragment de restriction recherché par exemple par électrophorèse préparative.

En vue de l'insertion du fragment cloné dans un vecteur permettant la transmission et le maintien de gènes étrangers dans des animaux transgéniques, on sépare avantageusement l'une ou les deux extrémités afin de pouvoir les coller à d'autres fragments et, le cas échéant, on ajoute des sites de restriction artificiels.

Le fragment d'ADN cloné est ensuite associé à un ou plusieurs gènes et à une séquence vectrice donnée. On procède avantageusement à une amplification, à chaque étape intermédiaire, dans un vecteur bactérien approprié.

Le vecteur bactérien comportant de tels clones constitue un produit nouveau et à ce titre fait également partie de l'invention.

Comme indiqué ci-dessus, l'étude par les inventeurs des fragments d'ADN comprenant ou correspondant à la séquence cen a mis en évidence leurs propriétés vis-à-vis de la replication autonome d'un vecteur les renfermant et/ou de la ségrégation mitotique et méiotique.

Ces propriétés sont mises à profit conformément à l'invention pour l'élaboration de vecteurs de microinjection, afin d'assurer le transfert et le maintien à l'état de vecteur circulaire de gènes étrangers dans des animaux transgéniques.

Contrairement aux techniques utilisées à ce jour, le transfert de gène étranger chez l'animal transgénique est réalisé sous forme d'un vecteur circulaire qui se maintient à l'état autonome.

Le vecteur navette défini comme ci-dessus, avec un gène inséré, peut être introduit dans la lignée germinale de l'espèce animale à modifier par microinjection de l'ADN recombinant, soit seul, soit en mélange avec un ADN codant pour la protéine grand T d'un Polyomavirus. On pourra aussi utiliser avantageusement les vecteurs de gènes dérivés de Retrovirus pour en assurer le transfert dans les cellules embryonnaires.

Les avantages apportés par de tels vecteurs sont considérables. Ils permettent en effet de supprimer les remaniements importants qu'occasionne l'intégration de gènes étrangers, le maintien des séquences injectées dans un environnement génétique contrôle, l'obtention d'un taux constant de transcription indépendant du site d'intégration.

Parmi les applications en tant que vecteurs de microinjection, on mentionnera le clonage de séquences cen à partir de molécules recombinantes produites in vivo après transfert des gènes précoces des polyomavirus et le clonage à partir de l'ADN d'une espèce par exemple autre que la souris, de molécules structuralement et fonctionnellement homologues des précédentes et identifiées par hybridation moléculaire avec celles-ci.

7

D'autres caractéristiques et avantages de l'invention apparaîtront dans les exemples qui suivents relatifs à des constructions plasmidiques renfermant la séquence cen.

EXEMPLE 1 .

- Construction d'un vecteur bactérien renfermant la séquence cen :

- on isole un fragment PvuII-HindII contenant les séquences centromériques de souris par digestion par les endonucléases PvuII et HindII du plasmide de souris p12B1, puis on purifie sur gel d'agarose 1% par électro-élution,

- on répare les extrémités du fragment par l'enzyme de modification "Mung-Bean-Nucléase", puis on clone le fragment au site Sana I du vecteur bactérien pUC18 par ligation entre extrémités franches à l'aide de la ligase du bacténophage T4.

- On procède à une transfection du mélange de réaction dans la souche E.coli JM105 et on étale sur un milieu gelosé en présence d'ampicilline, d'IPTG (isopopyl-β-D-thio-galactopyranoside) et de X-GAL (5-bromo-4-chloro-3-indolyl-β-galactoside).

- On sélectionne des clones recombinants (coloriés blanches) et on amplifie en culture liquide ; on purifie ensuite le plasmide recombinant suivant les techniques classiques.

EXEMPLE 2.

- Vecteur comportant une séquence cen - associée à des séquences d'ADN du gène HBS.

- On utilise le vecteur de l'exemple 1 dans lequel on a inséré les séquences d'ADN du gène HBs. (voir Y. Malpièce et al, dans Gene 1985 et Stenlund et al dans EMBO Journal, 2,669,1983). On constate que les séquences du gène HBs sont maintenues dans le vecteur dans le mouton.

8

EXEMPLE 3.

- Utilisation du plasmide de souris p12B1 comme vecteur propre au clonage de séquences étrangères.

Le plasmide P12B1 est isolé de la descendance d'une souche fondatrice obtenue par microinjection du plasmide pPyLT1 dans le pronucléus mâle d'oeufs fécondés. (Pour les techniques de microinjection, on peut se reporter à Hammer et al dans Nature, 315,680-683).

a/ Sites de clonage utilisés :

- il s'agit soit des sites existants, dans la mesure où ils ne compromettent ni le maintien à l'état autonome dans l'animal, ni la sélection après retour dans la bactérie, soit de sites artificiels : on ajoute alors une séquence portant un nombre défini de sites derestriction unique, dans une région inoffensive du vecteur.

b/ Sélection des recombinants :

- on effectue une hybridation in situ après clonage dans une région conservant intacte l'expression de la β-lactamase.

- on utilise un deuxième gène de sélection bactérien inséré dans le plasmide (Kana$^R$, Tetra$^R$, gène suppresseur) et on effectue une sélection négative.

- on utilise un système de sélection positive après insertion du gène lacZ équipé d'une séquence "polylinker" (dérivé des séquences de sélection présentes dans les vecteurs de type M13).

c/ Injection dans l'oeuf fécondé :

Les plasmides recombinants portant des séquences étrangères sont injectés dans l'oeuf fécondé de souris en utilisant les techniques conventionnelles de microinjection.

d/ Utilisation des vecteurs rétroviraux :

Le clonage des séquences ori-cen de souris est effectué dans un vecteur de type rétrovirus. On obtient

9

des animaux transgéniques par infection des oeufs fécondés.

e/ Sélection des animaux transgéniques et retour dans la bactérie :

Les animaux transgéniques sont identifiés par analyse de l'ADN de queue en hybridation avec la sonde correspondant au plasmide microinjecté. Le retour dans la bactérie des séquences microinjectées est rendu possible par simple transfection de l'ADN total dans un hôte bactérien du fait de la présence de ces séquences sous forme plasmidique et de leur sélection possible sur la base d'une résistance à un antibiotique.

EXEMPLE 4 :

- Application à la construction de vecteurs de microinjection dans d'autres espèces de mammifères.

1. Recherche de séquences ori-cen homologues.

L'utilisation par exemple de plasmides de souris comme sondes permet le criblage de banques géno-miques de l'espèce donnée et l'identification d'un clone ori-cen.

2. Test in vivo de recherche de la séquence ori-cen minimale.

a/ on effectue une sélection directe dans l'animal après microinjection d'un clone ori-cen entier inséré dans un vecteur de type pBR322.

b/ on réalise une microinjection de différentes délétions de la séquence ori-cen clonées dans un vecteur de type pBR322 et on détermine la séquence minimale responsable du maintien à l'état autonome, ce qui permet une analyse de cette séquence.

10

## REVENDICATIONS

1/ Fragments d'ADN caractérisés en ce qu'ils renferment une séquence de nucléotides à fonction centromérique, ou séquence cen, capable d'assurer la ségrégation régulière lors des mitoses ou des méioses associées à des séquences ori nécessaires à la réplication à l'état autonome.

2/ Fragments d'ADN du type de ceux obtenus selon la revendication 1 par clonage de l'ADN cellulaire sur la base de leur homologie avec les séquences de souris à fonction centromérique de vertébrés, notamment d'animaux d'élevage.

3/ Fragments d'ADN comprenant ou correspondant à des fragments d'ADN tels qu'obtenus par transposition d'ADN cellulaire de souris, dans un vecteur circulaire à la suite de l'expression d'un antigène T de Polyomavirus 4/ Fragments d'ADN selon l'une quelconque des revendications 1 à 3, caractérisés en ce qu'ils comportent à une ou aux deux extrémités des sites de restriction naturels et/ou artificiellement ajoutés.

5/ Vecteurs circulaires autonomes comprenant un fragment d'ADN selon l'une quelconque des revendications 1 à 4 associés à un ou plusieurs gènes à intérêt économique ou scientifique, et une séquence vectrice.

6/ Procédé d'obtention de fragments d'ADN selon l'une quelconque des revendications 1 à 4, par clonage à partir de vecteurs circulaires tels qu'obtenus à la suite de l'expression d'un antigène T de Polyomavirus, le clonage comportant la définition d'une carte de restriction du vecteur, l'identification des fragments comportant les séquences cellulaires, par exemple par hybridation, la purification du fragment de restriction recherché, par exemple par électrophorèse, suivie de la réparation éventuelle d'une ou des deux extrémités et, le cas échéant, aux fins d'addition à une séquence vectrice, pour l'élaboration d'un vecteur selon la revendication 5, de sites de restriction artificiels, le fragment d'ADN étant alors associé à un ou plusieurs gènes.

7/ Application d'un vecteur selon la revendication 5, comme vecteur de microinjection pour le clonage à partir de molécules recombinantes produites in vivo après transfert des gènes précoces des Polyomavirus, ou le clonage à partir de l'ADN d'une autre espèce que la souris, de molécules structuralement et fonctionnellement homologues des précédentes et identifiées par hybridation moléculaire avec celles-ci.

Office européen
des brevets

**RAPPORT DE RECHERCHE EUROPEENNE**

**0240373**
Numero de la demande

EP 87 40 0206

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | PROCEEDINGS OF THE NATIONAL ACADEMY OF SCIENCE, USA, vol. 81, août 1984, pages 4884-4888; E.W.JABS et al.: "Characterization of a cloned DNA sequence that is present at centromeres of all human autosomes and the X chromosome and shows polymorphic variation" * En entier * | 1,2,4, 5 | C 12 N 15/00 |
| X | PROCEEDINGS OF THE NATIONAL ACADEMY OF SCIENCES, USA, vol. 79, novembre 1982, pages 6593-6597; T.P.YANG et al.: "Characterization of a cloned repetitive DNA sequence concentrated on the human X chromosome" * Page 6593: abrégé * | 1,2,4, 5 | |
| X | EP-A-0 048 081 (THE REGENTS OF THE UNIVERSITY OF CALIFORNIA) * Abrégé * | 1,2,5 | **DOMAINES TECHNIQUES RECHERCHES (Int. Cl.4)** |
| X | GENE, vol. 34, 1984, pages 179-186, Elsevier Science Publishers; C.C.MEDDLE et al.: "Cloning of the CDC7 gene of Saccharomyces cerevisiae in association with centromeric DNA" * Page 179: abrégé * | 1,2,5 | C 12 N |

Le présent rapport de recherche a été établi pour toutes les revendications

| Lieu de la recherche | Date d'achèvement de la recherche | Examinateur |
|---|---|---|
| LA HAYE | 29-05-1987 | CUPIDO M. |